# EUROPEAN PATENT SPECIFICATION

(11) **EP 1 261 353 B1**
(45) Date of publication and mention of the grant of the patent: **28.07.2010**
(21) Application number: 01916582.8
(22) Date of filing: 12.03.2001
(51) Int. Cl.: A61K 31/711, A61P 31/20, C07H 21/00

(54) **IMMUNOSTIMULATORY POLYNUCLEOTIDE SEQUENCES FOR USE IN PREVENTING AND TREATING VIRAL INFECTIONS**
IMMUNOSTIMULIERENDE POLYNUKLEOTIDE SEQUENZEN ZUR VERWENDUNG BEI DER VERHINDERUNG UND BEHANDLUNG VON VIRALEN ERKRANKUNGEN
METHODES PERMETTANT DE REDUIRE L'INFECTION PAR PAPILLOMAVIRUS AU MOYEN DE SEQUENCES POLYNUCLEOTIDIQUES IMMUNOMODULATOIRES

(30) Priority: 10.03.2000 US 188265 P; 09.03.2001 US 802445
(43) Date of publication of application: 04.12.2002
(73) Proprietor: Dynavax Technologies Corporation, Berkeley, CA 94710 (US)
(72) Inventor: VAN NEST, Gary, Martinez, CA 94553 (US); EIDEN, Joseph J., Jr., Danville, CA 94506 (US)
(74) Representative: Roques, Sarah Elizabeth
(86) International application number: PCT/US2001/007842
(87) International publication number: WO 2001/068117

(56) References cited:
- WO-A2-98/55495
- WO-A2-99/51259
- MARTIN-OROZCO E ET AL: "ENHANCEMENT OF ANTIGEN-PRESENTING CELL SURFACE MOLECULES INVOLVED IN COGNATE INTERACTIONS BY IMMUNOSTIMULATORY DNA SEQUENCES" INTERNATIONAL IMMUNOLOGY, OXFORD UNIVERSITY PRESS, GB, vol. 11, no. 7, 1999, pages 111-118, XP000938271 ISSN: 0953-8178
- ROMAN MARK ET AL: "Immunostimulatory DNA sequences function as T helper-1-promoting adjuvants" NATURE MEDICINE, NATURE PUBLISHING, CO, US, vol. 3, no. 8, August 1997 (1997-08), pages 849-854, XP002188113 ISSN: 1078-8956
- FEARON K. ET AL: "A minimal human immunostimulatory CpG motif that potently induces IFN-Gamma and IFN-Alpha production" EUROPEAN JOURNAL OF IMMUNOLOGY, vol. 33, 1 January 2003 (2003-01-01), pages 2114-2122, XP007902578 WILEY - V C H VERLAG GMBH & CO. KGAA, DE

## Description

### CROSS-REFERENCE TO RELATED APPLICATIONS

This application claims the priority benefit of U.S. Provisional application 60/188,265, filed March 10, 2000.

### STATEMENT OF RIGHTS TO INVENTIONS MADE UNDER

### FEDERALLY SPONSORED RESEARCH

Experimental work described herein was performed at the National Institutes of Health (NCI and NIAID divisions). The Government may have certain rights in this invention.

### TECHNICAL FIELD

This invention is in the field of immunomodulatory polynucleotides, more particularly their use in ameliorating or preventing papillomavirus infection and/or symptoms of papillomavirus infections.

### BACKGROUND ART

Infection with human papillomavirus (HPV) is one of the most common sexually transmitted diseases (STD) in the United States. Over 100 types of HPV have been isolated and are categorized into two groups: cutaneous HPV and mucosal HPV. Cutaneous HPVs include more than 15 types of HPVs that are associated with different types of skin warts. Mucosal HPVs include more than 25 types of HPVs whose main reservoir is the genital tract. Other sites for mucosal HPVs include the respiratory tract and the oral cavity.

HPVs usually cause benign warts, or papillomas, that persist for several months to years. In some cases, the growth of warts may become life-threatening, for example, in the respiratory tract. In other cases, warts cause discomfort, pain, hoarseness of voice, perceived cosmetic flaws and may serve as a source of virus for sexual transmission of HPV.

Some types of mucosal HPVs (*e.g*., HPV-16, HPV-18, HPV-31 and HPV-45) are strongly associated with the development of cervical cancer. Cervical cancer is the second most common cancer among women worldwide. Annually, approximately 450,000 new cases are diagnosed and almost 200,000 deaths are due to cervical cancer. Pisani et al. (1993) Int. J. Cancer 55: 891-903. The overall 5-year survival rate is about 60%; however, survival rates can range from 15%, 35%, 65%, to 85% depending on how advanced the cancer is when the patient is diagnosed. Murakami et al. (1999) J. Immunother. 22(3): 212-218.

Current clinical treatments for warts include the application of caustic agents (tricholoracetic acid, podophyllin, podofilox, etc.), cryotherapy, immune modulators (intralesional interferon-α, topical Imiquimod, etc.), surgical therapy, and/or application of an inhibitor of DNA synthesis, such as 5-fluorouracil. Other treatments for warts utilize VLP (Virus-Like Particles). VLPs are immunologically active particles comprised of L1 and L2 capsid proteins synthesized using yeast or bacterial vectors or recombinant techniques. VLP-based vaccine studies in dogs, cattle, and rabbits have had success in wart regression (Hines et. al. (1996) Curr. Opin. Obstet. Gynecol. 10: 15-19); however, successful results have not yet been seen with VLP-based vaccines in humans. This approach, while feasible, does not account for physiologically relevant features of an intact, infectious virion. Features such as viral surface conformation, antigenicity of viral oncogenes such as E6 and E7 not present in VLP, and other non-expressed proteins that may serve as potential antigens all provide reasonable targets for the immune system. The aforementioned methods eliminate warts temporarily but cannot guarantee that recurrences of warts will not occur. Furthermore, removing warts temporarily does not eradicate the HPV causing the symptoms or address other aspects of infection, such as the possible progression of pre-neoplastic, HPV-infected cells to cancer.

In both humans and animals, benign papillomas caused by some types of HPV and animal papillomaviruses can progress to malignant cancers. The participation of co-factors, such as exposure to sunlight or x-irradiation, appear to be required for progression to malignancy in some HPV infections (*e.g*., HPV-5, HPV-6, HPV-8, and/or HPV-11). In animals, co-factors can include exposure to bracken fern (cattle), sunlight (sheep) and coal tar (rabbits). Co-factors that may may be associated with transformation of pre-neoplastic, HPV-infected cells to cervical cancer are still unknown but may include smoking, oral contraceptive use, pregnancy, other STDs, nutrition, immune status, and major histocompatibility complex (MHC) haplotypes.

Over 90% of cervical cancer are associated with high-grade HPV, such as HPV-16, HPV-18, and HPV-31. Bosch et al. (1995) J. Natl. Cancer Inst. 87:796-802. One possible cause of their high-risk potential is integration of the high-risk HPV viral genome into the host genome. The viral genome usually remains extra-chromosomal with low-risk HPV. Low-risk HPV types tend to cause more warts than high-risk HPV types and low-risk HPV types are not as strongly associated with cancer as the high-risk HPV types.

When the transformation of pre-neoplastic, HPV-infected cells to abnormal growth of cervical epithelium occurs, the abnormal growth is classified in two stages: low-grade squamous intraepithelial lesions (SIL) and high-grade SIL. Because the factors that are required for the onset of low grade SIL to occur are still unknown, a method of treatment that could prevent or delay the progression of a pre-neoplastic, HPV-infected cell to a carcinoma is highly desirable. To date, there is no known cure for cervical carcinoma. Chemotherapy is usually offered as therapy but does not guarantee that all carcinoma will be eradicated and has numerous side effects.

There is a need for a method of treatment would have one or more of the following desirable characteristics: the ability to boost anti-viral cell-mediated immune response, preferably Th1-based response; capacity to reduce or eradicate warts in humans and animals; reduce or eradicate both HPV and animal papillomavirus infection; treat disease symptoms stemming from papillomavirus infections; prevent and/or reduce the incidence of the recurring of these symptoms; prevent and/or reduce the incidence of progression of pre-neoplastic papillomavirus-infected cells to carcinoma; be generally applicable to multiple types of human papillomavirus as well as animal papillomaviruses.

Administration of certain DNA sequences, generally known as immunostimulatory sequences or "ISS," induces an immune response with a Th1-type bias as indicated by secretion of Th1-associated cytokines. The Th1 subset of helper cells is responsible for classical cell-mediated functions such as delayed-type hypersensitivity and activation of cytotoxic T lymphocytes (CTLs), whereas the Th2 subset functions more effectively as a helper for B-cell activation. The type of immune response to an antigen is generally influenced by the cytokines produced by the cells responding to the antigen. Differences in the cytokines secreted by Th1 and Th2 cells are believed to reflect different biological functions of these two subsets. See, for example, Romagnani (2000) Ann. Allergy. Asthma Immunol. 85:9-18.

Administration of an immunostimulatory polynucleotide with an antigen results in a Th1-type immune response to the administered antigen. Roman et al. (1997) Nature Med. 3:849-854. For example, mice injected intradermally with *Escherichia coli (E. coli)* β-galactosidase (β-gal) in saline or in the adjuvant alum responded by producing specific IgG1 and IgE antibodies, and CD4⁺ cells that secreted IL-4 and IL-5, but not IFN-γ, demonstrating that the T cells were predominantly of the Th2 subset. However, mice injected intradermally (or with a tyne skin scratch applicator) with plasmid DNA (in saline) encoding β-Gal and containing an ISS responded by producing IgG2a antibodies and CD4⁺ cells that secreted IFN-γ, but not IL-4 and IL-5, demonstrating that the T cells were predominantly of the Th1 subset. Moreover, specific IgE production by the plasmid DNA-injected mice was reduced 66-75%. Raz et al. (1996) Proc. Natl. Acad Sci. USA 93:5141-5145. In general, the response to naked DNA immunization is characterized by production of IL-2, TNFα and IFN-γ by antigen-stimulated CD4⁺ T cells, which is indicative of a Th1-type response. This is particularly important in treatment of allergy and asthma as shown by the decreased IgE production. The ability of immunostimulatory polynucleotides to stimulate a Th1-type immune response has been demonstrated with bacterial antigens, viral antigens and with allergens (see, for example, WO 98/55495).

Other references describing ISS include: Krieg et al. (1989) J. Immunol. 143:2448-2451; Tokunaga et al. (1992) Microbiol. Immunol. 36:55-66; Kataoka et al. (1992) Jpn. J. Cancer Res. 83:244-247; Yamamoto et al. (1992) J. Immunol. 148:4072-4076; Mojcik et al. (1993) Clin. Immuno. and Immunopathol. 67:130-136; Branda et al. (1993) Biochem. Pharmacol. 45:2037-2043; Pisetsky et al. (1994) Life Sci. 54(2):101-107; Yamamoto et al. (1994a) Antisense Research and Development. 4:119-122; Yamamoto et al. (1994b) Jpn. J. Cancer Res. 85:775-779; Raz et al. (1994) Proc. Natl. Acad. Sci. USA 91:9519-9523; Kimura et al. (1994) J. Biochem. (Tokyo) 116:991-994; Krieg et al. (1995) Nature 374:546-549; Pisetsky et al. (1995) Ann. N.Y. Acad. Sci. 772:152-163; Pisetsky (1996a) J. Immunol. 156:421-423; Pisetsky (1996b) Immunity 5:303-310; Zhao et al. (1996) Biochem. Pharmacol. 51:173-182; Yi et al. (1996) J. Immunol. 156:558-564; Krieg (1996) Trends Microbiol. 4(2):73-76; Krieg et al. (1996) Antisense Nucleic Acid Drug Dev. 6:133-139; Klinman et al. (1996) Proc. Natl. Acad. Sci. USA. 93:2879-2883; Raz et al. (1996); Sato et al. (1996) Science 273:352-354; Stacey et al. (1996) J. Immunol. 157:2116-2122; Ballas et al. (1996) J. Immunol. 157:1840-1845; Branda et al. (1996) J. Lab. Clin. Med. 128:329-338; Sonehara et al. (1996) J. Interferon and Cytokine Res. 16:799-803; Klinman et al. (1997) J. Immunol. 158:3635-3639; Sparwasser et al. (1997) Eur. J. Immunol. 27:1671-1679; Roman et al. (1997); Carson et al. (1997) J. Exp. Med. 186:1621-1622; Chace et al. (1997) Clin. Immunol. and Immunopathol. 84:185-193; Chu et al. (1997) J. Exp. Med. 186:1623-1631; Lipford et al. (1997a) Eur. J. Immunol. 27:2340-2344; Lipford et al. (1997b) Eur. J. Immunol. 27:3420-3426; Weiner et al. (1997) Proc. Natl. Acad. Sci. USA 94:10833-10837; Macfarlane et al. (1997) Immunology 91:586-593; Schwartz et al. (1997) J. Clin. Invest. 100:68-73; Stein et al. (1997) Antisense Technology, Ch. 11 pp. 241-264, C. Lichtenstein and W. Nellen, Eds., IRL Press; Wooldridge et al. (1997) Blood 89:2994-2998; Leclerc et al. (1997) Cell. Immunol. 179:97-106; Kline et al. (1997) J. Invest. Med. 45(3):282A; Yi et al. (1998a) J. Immunol. 160:1240-1245; Yi et al. (1998b) J. Immunol. 160:4755-4761; Yi et al. (1998c) J. Immunol. 160:5898-5906; Yi et al. (1998d) J. Immunol. 161:4493-4497; Krieg (1998) Applied Antisense Oligonucleotide Technology Ch. 24, pp. 431-448, C.A. Stein and A.M. Krieg, Eds., Wiley-Liss, Inc.; Krieg et al. (1998a) Trends Microbiol. 6:23-27; Krieg et al. (1998b) J. Immunol. 161:2428-2434; Krieg et al. (1998c) Proc. Natl. Acad. Sci. USA 95:12631-12636; Spiegelberg et al. (1998) Allergy 53(45S):93-97; Homer et al. (1998) Cell Immunol. 190:77-82; Jakob et al. (1998) J. Immunol. 161:3042-3049; Redford et al. (1998) J. Immunol. 161:3930-3935; Weeratna et al. (1998) Antisense & Nucleic Acid Drug Development 8:351-356; McCluskie et al. (1998) J. Immunol. 161(9):4463-4466; Gramzinski et al. (1998) Mol. Med. 4:109-118; Liu et al. (1998) Blood 92:3730-3736; Moldoveanu et al. (1998) Vaccine 16: 1216-1224; Brazolot Milan et al. (1998) Proc. Natl. Acad. Sci. USA 95:15553-15558; Broide et al. (1998) J. Immunol. 161:7054-7062; Broide et al. (1999) Int. Arch. Allergy Immunol. 118:453-456; Kovarik et al. (1999) J. Immunol. 162:1611-1617; Spiegelberg et al. (1999) Pediatr. Pulmonol. Suppl. 18:118-121; Martin-Orozco et al. (1999) Int. Immunol. 11:1111-1118; EP 468,520; WO 96/02555; WO 97/28259; WO 98/16247; WO 98/18810; WO 98/37919; WO 98/40100; WO 98/52581; WO 98/55495; WO 98/55609 and WO 99/11275. See also Elkins et al. (1999) J. Immunol. 162:2291-2298, WO 98/52962, WO 99/33488, WO 99/33868, WO 99/51259 and WO 99/62923. See also Zimmermann et al. (1998) J. Immunol. 160:3627-3630; Krieg (1999) Trends Microbiol. 7:64-65; U.S. Patent Nos. 5,663,153, 5,723,335, 5,849,719 and 6,174,872. See also WO99/56755, WO00/06588, WO00/16804; WO00/21556; WO00/67023 and WO01/12223.

There remains a serious need to develop effective therapies and preventive strategies for papillomaviruses.

### DISCLOSURE OF THE INVENTION

We describe methods of suppressing, ameliorating and/or preventing papillomavirus infection in an individual using immunostimulatory polynucleotide sequences. Accordingly, in one aspect, the invention provides a composition comprising a polynucleotide comprising an immunostimulatory sequence (ISS), for use in a method for treating a symptom of papillomavirus infection in an individual who has been exposed to papillomavirus, wherein the ISS comprises the sequence 5'-C,G,pyrimidine,pyrimidine,C,G-3' and the polynucleotide is 8 to 50 base or base pairs in length, wherein the polynucleotide is administered without co-administration of a papillomavirus antigen, wherein said composition does not comprise a cytokine and wherein said composition is for administration in an amount sufficient to treat a symptom of papillomavirus infection, and wherein said composition is to be administered at a site of a papilloma lesion.

We also describe methods of palliating, ameliorating, reducing severity, and/or eliminating one or more symptoms of *papillomavirinae* infection without *administering papillomavirinae* antigen. A polynucleotide comprising an immunostimulatory sequence (an "ISS") is administered to an individual who is at risk of being exposed to *papillomavirinae,* has been exposed to *papillomavirinae* and/or is infected with *papillomavirinae.* The ISS-containing polynucleotide is administered without any *papillomavirinae* antigen. Administration of the ISS results in reduced incidence, recurrence, and/or severity of one or more symptoms of *papillomavirinae* infection.

We describe methods for preventing a symptom of *papillomavirinae* infection in an individual at risk of being exposed to *papillomavirinae* which entail administering an effective amount of a composition comprising a polynucleotide comprising an immunostimulatory sequence (ISS) (*i.e.,* an amount of the composition sufficient to prevent a symptom of *papillomavirinae* infection) to the individual, wherein the ISS comprises the sequence 5'-C, G, pyrimidine, pyrimidine, C, G-3' and wherein a *papillomavirinae* antigen is not administered in conjunction with administration of the composition (*i.e*., antigen is not administered with the ISS-containing polynucleotide), thereby preventing a symptom of *papillomavirinae* infection.

We also describe methods for preventing a symptom of *papillomavirinae* infection in an individual which entail administering an effective amount of a composition comprising a polynucleotide comprising an ISS to the individual, wherein the ISS comprises the sequence 5'-C, G, pyrimidine, pyrimidine, C, G-3' and wherein a *papillomavirinae* antigen is not administered in conjunction with administration of the composition, thereby preventing a symptom of *papillomavirinae* infection. The individual may have been exposed m or infected by *papillomavtrinae.*

We describe methods for suppressing a *papillomavirinae* infection in an individual which entail administering an effective amount of a composition comprising a polynucleotide comprising an ISS to the individual, wherein the ISS comprises the sequence 5'-C, G, pyrimidine, pyrimidine, C, G-3' and wherein a *papillomavirinae* antigen is not administered in conjunction with admnistration of the composition, thereby suppressing a *papillomavirinae* information. The individual may have been exposed to or infected by *papillomavirinae.*

We also describe methods of reducing seventy of a symptom of *papillomavirinae* infection in an individual which entail administering an effective amount of a composition composing a polynucleotide comprising an ISS to the individual, wherein the ISS comprises the sequence 5'-C, G, pyrimidine, pyrimidine, C, G-3' and wherein a *papillomavirinae* antigen is not administered in conjunction with administration of the composition, thereby reducing severity of a symptom of *papillomavirinae* infection. The individual may have been exposed to or infected by *papillomavirinae*.

We describe methods of delaying development of a *papillomavirinae* infection and/or a symptom of *papillomavirinae* infection in an individual which entail administering an effective amount of a composition comprising a polynucleotide comprising an ISS to the individual, wherein the ISS comprises the sequence 5'-C, G, pyrimidine, pyrimidine, C, G-3' and wherein a *papillomavirinae* antigen is not administered in conjunction with administration of the composition, thereby delaying development of a *papillomavirinae* infection and/or a symptom of *papillomavirinae* infection. The individual may have been exposed to or infected by *papillomavirinae.*

We also describe methods of reducing duration of a *papillomavirinae* infection in an individual which entail administering an effective amount of a composition comprising a polynucleotide comprising an ISS to the individual, wherein the ISS comprises the sequence 5'-C, G, pyrimidine, pyrimidine, C, G-3' and wherein a *papillomavirinae* antigen is not administered in conjunction with administration of the composition, thereby reducing duration of a infection. The individual may have been exposed to or infected by *papillomavirinae.*

We describe methods of reducing recurrence of a symptom of *papillomavirinae* infection in an individual infected with *papillomavirinae* which entail administering an effective amount of a composition comprising a polynucleotide comprising an ISS to the individual, wherein the ISS comprises the sequence 5'-C, G, pyrimidme, pyrimidme, C, G-3' and wherein a *papillomavirinae* antigen is not administered in conjunction with administration of the composition, thereby reducing recurrence of a symptom of *papillomavirinae* infection.

We describe methods for suppressing papillomavirus infection in a papillomavirus-infected individual which entail administering an effective amount of a composition comprising a polynucleotide comprising an ISS to the individual, wherein the ISS comprises the sequence 5'-C, G, pyrimidine, pyrimidine, C, G-3' and wherein a *papillomavirinae* antigen is not administered in conjunction with administration of the composition, thereby suppressing papillomavirus infection.

We also describe kits for use in ameliorating and/or preventing a symptom of *papillomavirinae* infection in an individual infected with, exposed to or at risk of being exposed to *papillomavirinae* and/or reduction in recurrence of a symptom of *papillomavirinae* infection. The kits comprises a composition comprising a polynucleotide comprising an ISS, wherein the ISS comprises the sequence 5'-C, G, pyrimidine, pyrimidine, C, G-3' and wherein the kit does not comprise a *papillomavirinae* antigen, and wherein the kits comprise instructions for administration of the composition to an individual infected with, exposed to or at risk of being exposed to *papillomavirinae.*

In some embodiments of the methods and kits of the invention, the ISS comprises the sequence 5' purine, purine, C, G, pyrimidine, pyrimidine, C, G-3'. In further embodiments of the methods and kits, the ISS comprises a sequence selected from the group consisting of AACGTTCG and GACGTTCG.

In some embodiments of the methods and kits of the invention, the ISS comprises the sequence 5'-TGACTGTGAACGITCGAGATGA-3'(SEQ ID NO:1).

In some embodiments of the methods and kits of the invention, the individual is a mammal. In further embodiments, the mammal is human.

In some embodiments of the methods and kits of the invention, the *papillomavirinae* is a papillomavirus. In further embodiments of the methods and kits of the invention, the papillomavirus is a human papillomavirus (HPV) or an animal papillomavirus.

### BRIEF DESCRIPTION OF THE DRAWINGS

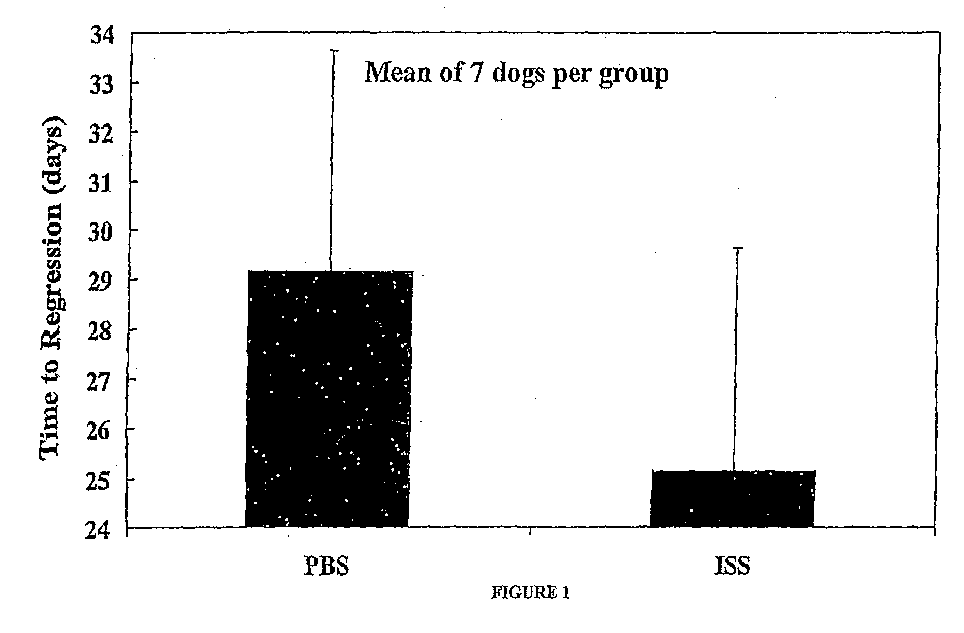

Figure 1 is a bar graph depicting results of ISS treatment in a canine model of papillomavirus for time of wart regression.

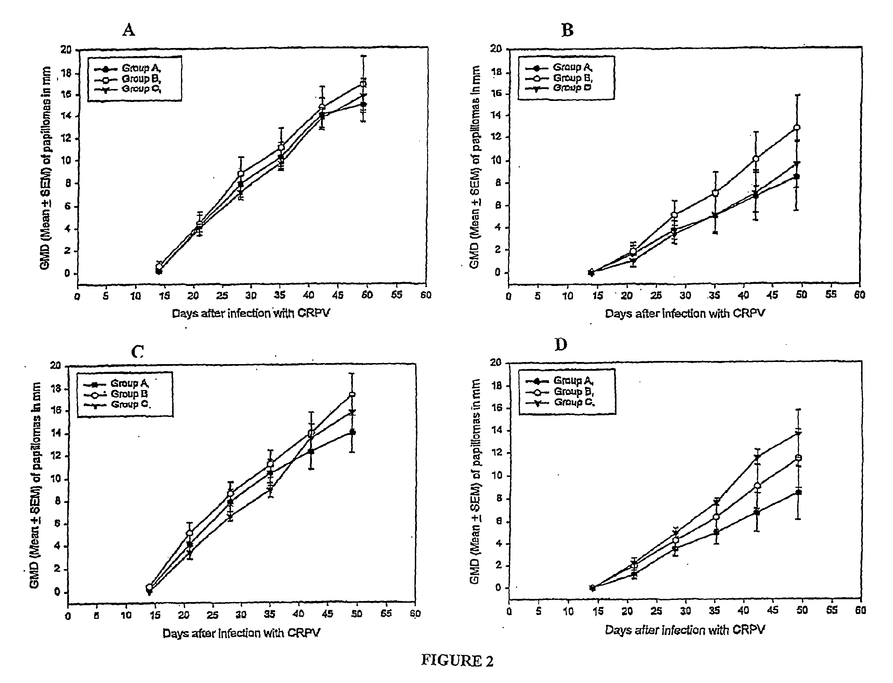

Figure 2(A) - (D) are graphs depicting results of ISS treatment of papillomavirus in a rabbit model. The data is expressed as geometric mean diameter (GMD) over time after inoculation. Closed circles indicate Group A animals, open circles indicate Group B animals, and closed triangles indicate Group C animals. Fig. 2(A) depicts GMD for the left side, high CRPV dose lesions. Fig. 2(B) depicts GMD for the left side, low CRPV dose lesions. Fig. 2(C) depicts average GMD for the right side, high CRPV dose lesions. Fig. 2(D) depicts average GMD for the right side, low CRPV dose lesions.

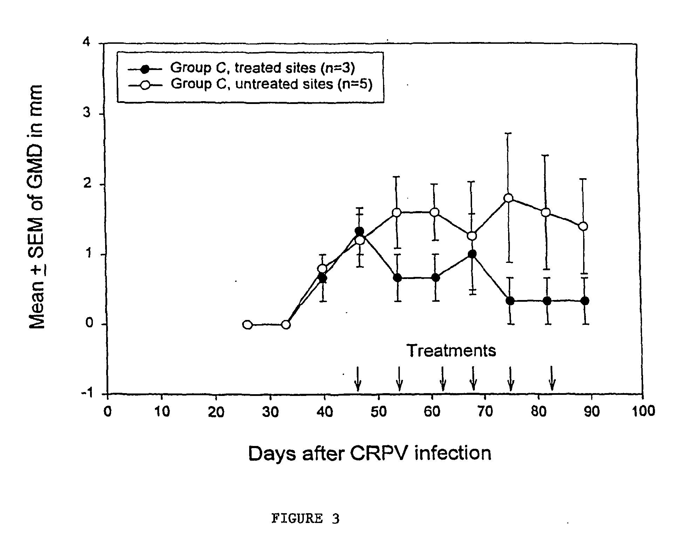

Figure 3 is a graph depicting results of ISS treatment of rabbit papillomavirus. The data is expressed as geometric mean diameter (GMD) over time after inoculation. Closed circles indicate ISS treated papilloma sites, open circles indicate untreated papilloma sites animals, and downward arrows indicate timing of ISS treatments.

### MODES FOR CARRYING OUT THE INVENTION

We have discovered methods of treating papillomavirus infections. The methods described herein are applicable to all *papillomavirinae* and particularly to methods of ameliorating infection (including recurrences) with a member of the *papillomavirinae* subfamily, preferably papillomavirus (including high risk and low risk). A polynucleotide comprising an immunostimulatory sequence (an "ISS") is administered to an individual who has been exposed to and/or infected with *papillomavirinae.* Administration of the ISS-containing polynucleotide without co-administration of a papillomavirus antigen results in reduced severity of one or more symptoms of *papillomavirinae* infection. It is understood that all the embodiments describes herein do not include or involve papillomavirus antigen.

The invention also relates to kits for ameliorating and/or preventing *papillomavirinae* infection and/or a symptom of *papillomavirinae* infection in exposed individuals. The kits, which do not contain an *papillomavirinae* antigen, comprise a polynucleotide comprising an ISS and instructions describing the administration of an ISS-containing polynucleotide to an individual for the intended treatment.

### General techniques

The practice of the present invention will employ, unless otherwise indicated, conventional techniques of molecular biology (including recombinant techniques), microbiology, cell biology, biochemistry and immunology, which are within the skill of the art. Such techniques are explained fully in the literature, such as, Molecular Cloning: A Laboratory Manual, second edition (Sambrook et al., 1989); Oligonucleotide Synthesis (M.J. Gait, ed., 1984); Animal Cell Culture (R.I. Freshney, ed., 1987); Handbook of Experimental Immunology (D.M. Weir & C.C. Blackwell, eds.); Gene Transfer Vectors for Mammalian Cells (J.M. Miller & M.P. Calos, eds., 1987); Current Protocols in Molecular Biology (F.M. Ausubel et al., eds., 1987); PCR: The Polymerase Chain Reaction, (Mullis et al., eds., 1994); Current Protocols in Immunology (J.E. Coligan et al., eds., 1991); The Immunoassay Handbook (David Wild, ed., Stockton Press NY, 1994); and Methods of Immunological Analysis (R. Masseyeff, W.H. Albert, and N.A. Staines, eds., Weinheim: VCH Verlags gesellschaft mbH, 1993).

### Definitions

"Papillomavirus" refers to a type of virus that is in the subfamily *papillomavirinae.* Members of *papillomavirinae* are sometimes grouped within a larger family of *papovaviridae,* which includes not only the papillomaviruses, but also polyomaviruses and simian vacuolating virus. Papillomaviruses are small, nonenveloped viruses with an icosahedral symmetry, 72 capsomers, and a double-stranded DNA genome of about 8,000 base pairs. There are about ten open reading frames (ORF) and all ORFs are located on one strand. The papillomavirus genome is organized into an early region and a late region. Early region genes code for proteins (E1-E8) required for viral DNA replication and cellular transformation while the late genes code for capsid protein (L1 and L2) and a regulatory region of transcriptional and replication control.

The term "human papillomavirus" ("HPV") refers to papillomaviruses of human species origin and/or which are capable of infecting a human. There are over 100 types of HPV. HPV can be divided into cutaneous HPV or mucosal HPV, depending on location where the pathology and/or infection occurs. HPV can additionally be divided into "high-risk" and "low-risk".

The term "high-risk" or "high-grade"refers to HPV that are strongly associated with cellular transformations that may lead to neoplasia and/or carcinoma. HPV types that are associated with development of carcinoma include, but are not limited to, HPV-16, -18, - 30, -31, -33, -34, -35, -39, -40, -41, -42, -43, -44, -45, -51, -52, -56, -57, -58, -61, -62, -66, - 69.

The term "low-risk" refers to HPV types that have lower cellular transformation potentials including, but not limited to, HPV-6 and HPV-11.

The term "animal papillomavirus" refers to papillomaviruses of non-human species origin, including, but is not limited to, cattle, horses, deer, rabbits, sheep, dogs, elk, nonhuman primates, rodents, harvest mice, multimammate mice, parrots and chaffinches.

The term "papilloma" herein refers to papillomavirus-associated warts, the development, appearance, keratinous texture and histological features thereof.

The term "condyloma" herein refers to an HPV-associated wart usually seen on the external genitalia or near the anus.

"Exposure" to a virus denotes encounter with virus which allows infection, such as, for example, surface to surface contact with an infected individual or tissue that results in minor trauma of basal cells and opportunity for papillomavirus to infect the traumatized basal cells, including, but are not limited to, non-barrier sexual contact and/or intercourse with an individual with infected genitalia and abrasion of skin epithelium with infectious tissue as in the case of butcher handling infected meat products.

An individual is "seronegative" for a virus if antibodies specific to the virus cannot be detected in blood or serum samples from the individual using methods standard in the art, such as ELISA. Conversely, an individual is "seropositive" for a virus if antibodies specific for the virus can be detected in blood or serum samples from the individual using methods standard in the art, such as ELISA. An individual is said to "seroconvert" for a virus when antibodies to the virus can be detected in blood or serum from an individual who was previously seronegative.

An individual who is "at risk of being exposed" to a papillomavirus is an individual who may encounter the virus such that the papillomavirus infects the individual (*i.e.,* virus enters cells and replicates). Because papillomaviruses are ubiquitous, generally any individual is at risk for exposure to papillomavirus. In some contexts, an individual is at risk for exposure of HPV by engaging in one or more high risk behaviors, such as sexual relations without the use of barrier prophylactics with an infected individual.

A "symptom of papillomavirus infection" is any one or more symptoms of papillomavirus infection and includes, but is not limited to, the clinical presentation of warts, condylomas and papillomas, all of which can be collectively referred to as "lesions". The term "symptoms of papillomavirus infection" also includes secondary symptoms associated with warts, condylomas, papillomas and lesions. These secondary symptoms can include, but are not limited to, hoarseness of voice, breathing difficulties, pain and discomfort.

"Preventing a symptom of infection" by a papillomavirus means that the symptom does not appear after exposure to the virus.

"Suppressing" papillomavirus infection indicates any aspect of viral infection, such as viral replication, time course of infection, amount (titer) of virus, lesions, and/or one or more symptoms is curtailed, inhibited, or reduced (in terms of severity and/or duration) in an individual or a population of individuals treated with an ISS-containing polynucleotide in accordance with the invention as compared to an aspect of viral infection in an individual or a population of individuals not treated in accordance with the invention. Reduction in viral titer includes, but is not limited to, elimination of the virus from an infected site or individual. Viral infection can be assessed by any means known in the art, including, but not limited to, measurement of virus particles, viral nucleic acid or viral antigens and detection of one or more symptoms of viral infection. Anti-virus antibodies are widely used to detect and monitor viral infection and generally are commercially available.

"Palliating" a disease or one or more symptoms of a disease or infection means lessening the extent and/or time course of undesirable clinical manifestations of a disease state or infection in an individual or population of individuals treated with an ISS in accordance with the invention.

As used herein, "delaying" development of a viral infection or a symptom of viral infection means to defer, hinder, slow, retard, stabilize, and/or postpone development of the disease or symptom when compared to not using the method(s) of the invention. This delay can be of varying lengths of time, depending on the history of the disease and/or individual being treated. As is evident to one skilled in the art, a sufficient or significant delay can, in effect, encompass prevention, in that the individual does not develop the disease.

"Reducing severity of a symptom" or "ameliorating a symptom" of viral infection means a lessening or improvement of one or more symptoms of viral infection as compared to not administering an ISS-containing polynucleotide. "Reducing severity" also includes shortening or reduction in duration of a symptom. For *papillomavirinae,* these symptoms are well known in the art and include, but are not limited to, the clinical presentation of warts, condyloma and papilloma.

"Reducing duration of viral infection" means the length of time of viral infection (usually indicated by symptoms) is reduced, or shortened, as compared to not administering an ISS-containing polynucleotide.

"Reducing recurrence" refers to a reduction in frequency, severity and/or quantity of one or more recurrent viral symptoms in an infected individual or a population of infected individuals. When applied to a population of individuals, "reducing recurrence" means a reduction in the mean or median frequency, severity, quantity and/or duration of recurrent viral symptoms.

The term "infected individual" refers to an individual who has been infected with a member of *papillomavirinae.* Symptoms of *papillomavirinae* infection are well known in the art and include, but are not limited to, the clinical presentation of warts, condyloma and papilloma. "Infected individual" can include asymptomatic, infected individuals. Identification of asymptomatic, infected individuals can be accomplished by any of the biological viral detection methods known in the art, which can include, but is not limited to, methods such as PCR*, in situ* hybridization and ELISA for virus-specific antibodies.

The term "eradicating papillomavirus infection" refers to elimination of the virus from the body of the infected individual. An implication from eradicating virus is that the immediate symptoms caused by the virus would also be eliminated, as well as certain events or conditions associated with viral infection.

The term "low-grade squamous intraepithelial lesion" (SIL) and "high-grade squamous intraepithelial lesion" (SIL) refers to the current classification scheme to describe abnormal growth of cervical cells. The category of low-grade SIL includes HPV infection and cervical intraepithelial neoplasia (CIN) 1 while high-grade SIL includes CIN 2 and 3 when the entire thickness of the epithelium is replaced by abnormal cells. This classification scheme is equivalent to a previous classification scheme in which the different stages ranged from cervical intraepithelial neoplasia (CIN) 1 (mild dysplasia) to CIN 2 (moderate dysplasia) to CIN 3 (carcinoma *in situ*).

A "biological sample" encompasses a variety of sample types obtained from an individual and can be used in a diagnostic or monitoring assay. The definition encompasses blood and other liquid samples of biological origin, solid tissue samples such as a biopsy specimen or tissue cultures or cells derived therefrom, and the progeny thereof. The definition also includes samples that have been manipulated in any way after their procurement, such as by treatment with reagents, solubilization, or enrichment for certain components, such as proteins or polynucleotides. The term "biological sample" encompasses a clinical sample, and also includes cells in culture, cell supernatants, cell lysates, serum, plasma, biological fluid, and tissue samples.

"Viral titer" is a term well known in the art and indicates the amount of virus in a given biological sample. Amount of virus are indicated by various measurements, including, but not limited to, amount of viral nucleic acid; presence of viral particles; replicating units (RU); plaque forming units (PFU). Generally, for fluid samples such as blood and urine, amount of virus is determined per unit fluid, such as milliliters. For solid samples such as tissue samples, amount of virus is determined per weight unit, such as grams. Methods for determining amount of virus are known in the art and described herein.

An "individual" is a vertebrate, preferably a mammal, more preferably a human. Mammals include, but are not limited to, humans, farm animals, sport animals, rodents, primates and certain pets. Vertebrates also include, but are not limited to, birds (i. e., avian individuals) and reptiles (*i.e.,* reptilian individuals).

The term "ISS" as used herein refers to polynucleotide sequences that effect a measurable immune response as measured *in vitro, in vivo* and/or *ex vivo.* Examples of measurable immune responses include, but are not limited to, antigen-specific antibody production, secretion of cytokines, activation or expansion of lymphocyte populations such as NK cells, CD4⁺ T lymphocytes, CD8⁺ T lymphocytes, B lymphocytes, and the like. Preferably, the ISS sequences preferentially activate a Th1-type response. A polynucleotide for use in methods of the invention contains at least one ISS.

As used interchangeably herein, the terms "polynucleotide" and "oligonucleotide" include single-stranded DNA (ssDNA), double-stranded DNA (dsDNA), single-stranded RNA (ssRNA) and double-stranded RNA (dsRNA), modified oligonucleotides and oligonucleosides or combinations thereof. The polynucleotide can be linearly or circularly configured, or the polynucleotide can contain both linear and circular segments.

"Adjuvant" refers to a substance which, when added to an immunogenic agent such as antigen, nonspecifically enhances or potentiates an immune response to the agent in the recipient host upon exposure to the mixture.

An "effective amount" or a "sufficient amount" of a substance is an amount sufficient to effect beneficial or desired results, including clinical results. An effective amount can be administered in one or more administrations. A "therapeutically effective amount" is an amount to effect beneficial clinical results, including, but not limited to, alleviation of one or more symptoms associated with viral infection as well as prevention of disease(*e.g*., prevention of one or more symptoms of infection).

A microcarrier is considered "biodegradable" if it is degradable or erodable under normal mammalian physiological conditions. Generally, a microcarrier is considered biodegradable if it is degraded (*i.e*., loses at least 5% of its mass and/or average polymer length) after a 72 hour incubation at 37° C in normal human serum. Conversely, a microcarrier is considered "nonbiodegradable" if it is not degraded or eroded under normal mammalian physiological conditions. Generally, a microcarrier is considered nonbiodegradable if it not degraded (*i.e.,* loses less than 5% of its mass and/or average polymer length) after at 72 hour incubation at 37° C in normal human serum.

The term "immunostimulatory sequence-microcarrier complex" or "ISS-MC complex" refers to a complex of an ISS-containing polynucleotide and a microcarrier. The components of the complex may be covalently or non-covalently linked. Non-covalent linkages may be mediated by any non-covalent bonding force, including by hydrophobic interaction, ionic (electrostatic) bonding, hydrogen bonds and/or van der Waals attractions. In the case of hydrophobic linkages, the linkage is generally via a hydrophobic moiety (*e.g*., cholesterol) covalently linked to the ISS.

As used herein, the term "comprising" and its cognates are used in their inclusive sense; that is, equivalent to the term "including" and its corresponding cognates.

As used herein, the singular form "a", "an", and "the" includes plural references unless indicated otherwise. For example, "a" symptom of viral infection includes one or more additional symptoms.

### Methods of the invention

The invention provides methods for preventing one or more symptoms of papillomavirus infection, treating, reducing severity of and/or delaying development of one or more symptoms of papillomavirus infection, treating and/or eradicating papillomavirus infection, reducing recurrence of one or more symptoms of papillomavirus infection by administering an ISS-containing polynucleotide (used interchangeably herein with "ISS") without a papillomavirus antigen. Papillomavirus can be any of the members of the *papillomavirinae* subfamily, preferably one or more of the HPV types or animal papillomaviruses of any type, including high-risk or low-risk types. An ISS-containing composition which does not include papillomavirus antigen is administered to individuals who are infected with papillomavirus, who have been exposed to papillomavirus or who are at risk of being exposed to papillomavirus. Individuals receiving ISS are preferably mammal, more preferably human. In accordance with the invention, papillomavirus antigen is not administered to the individual in conjunction with administration of an ISS *(i.e.,* is not administered in a separate administration at or about the same time of administration of the ISS).

In some embodiments, the individual is at risk of being exposed to virus. Determination of an at risk individual is based on one or more factors that are associated with disease development and are generally known by, or can be assessed by, a skilled clinician. At risk individuals may be especially suitable candidates to receive ISS, as these individuals are generally considered to be particularly susceptible to developing symptoms of infection, which could also further lead to other complications. For example, in the context of HPV infection, any individual is considered at risk, due to the wide spread prevalence of HPV infection. As another example, an individual at risk is an individual who practices unsafe sexual practices (*e.g*., engages in anal-genital or genital-genital contact without the use of barrier-type prophylactics).

In other embodiments, the individual is, or has been exposed to and/or infected with papillomavirus. Exposure can be indicated by participation in unsafe sexual practices and/or development of one or more symptoms associated with viral infection. For example, in dogs, oral warts may be construed as one symptom stemming from infection with canine papillomavirus. In another instance, in humans, skin warts may construed as one symptom stemming from infection with a type of HPV. The infected individual may or may not be symptomatic. Determination of infection can be based on any clinical indicia of infection, such as lesions or warts. Determination of infection in an asymptomatic individual can be accomplished by any of the methods known in the art, including, but not limited to, PCR techniques, *in situ* hybridization and ELISA for papillomavirus-specific antibodies. In other embodiments, the individual, preferably a human, is infected with papillomavirus, with or without symptoms stemming from infection, and may be at a stage prior to the development of carcinoma. Infection with papillomavirus can be ascertained by any of the aforementioned biological methods, while carcinoma may be detected with methods known in the art, including, but not limited to, Pap smears, biopsies, or other methods to observe morphological changes of epithelial cells.

### ISS

The methods of this invention entail administering an immunomodulatory polynucleotide comprising an ISS (or a composition comprising such a polynucleotide). In accordance with the present invention, the immunomodulatory polynucleotide contains at least one ISS, and may contain multiple ISSs. The ISSs may be adjacent within the polynucleotide, or they may be separated by additional nucleotide bases within the polynucleotide. Alternately, multiple ISSs may be delivered as individual polynucleotides.

ISS have been described in the art and may be readily identified using standard assays which indicate various aspects of the immune response, such as cytokine secretion, antibody production, NK cell activation and T cell proliferation. See, *e.g*., WO 97/28259; WO 98/16247; WO 99/11275; Krieg et al. (1995); Yamamoto et al. (1992); Ballas et al. (1996); Klinman et al. (1997); Sato et al. (1996); Pisetsky (1996a); Shimada et al. (1986) Jpn. J. Cancer Res. 77:808-816; Cowdery et al. (1996) J. Immunol. 156:4570-4575; Roman et al. (1997); and Lipford et al. (1997a).

The ISS can be of any length from 8 to 50 bases or base pairs and generally comprises the sequence 5'-cytosine, guanine-3', preferably greater than 15 bases or base pairs, more preferably greater than 20 bases or base pairs in length. As is well-known in the art, the cytosine of the 5'-cytosine, guanine-3' sequence is unmethylated. An ISS may also comprise the sequence 5'-purine, purine, C, G, pyrimidine, pyrimidine, C, G-3'- An ISS may also comprise the sequence 5'-purine, purine, C, G, pyrimidine, pyrimidine, C, C-3'. As indicated in polynucleotide sequences below, an ISS may comprise (*i.e*., contain one or more of) the sequence 5'-T, C, G-3'. In some embodiment, an ISS may comprise the sequence 5'-C, G, pyrimidine, pyrimidine, C, G-3' (such as 5'-CGTTCG-3'). In some embodiments, an ISS may comprise the sequence 5'-C, G, pyrimidine, pyrimidine, C, G, purine, purine-3'. In some embodiments, an ISS comprises the sequence 5'-purine, purine, C, G, pyrimidine, pyrimidine-3' (such as 5'-AACGTT-3').

In some embodiments, an ISS may comprise the sequence 5'-purine, T, C, G, pyrimidine, pynmidine-3'.

In some embodiments, an ISS-containing polynucleotide is less than about any of the following lengths (in bases or base pairs): 50; 25; 10. In some embodiments, an ISS-containing polynucleotide is greater than about any of the following lengths (in bases or base pairs): 8; 10; 15; 20; 25; 30; 40. Alternately, the ISS can be any of a range of sizes having an upper limit of 50; 25; or our 10 and an independently selected lower limit of 8; 10; 15; 20; 25; 30; wherein the lower limit is less than the upper limit.

In some embodiments, the ISS comprises any of the following sequences:
GACGCTCC; GACGTCCC; GACGTTCC; GACGCCCC; AGCGTTCC; AGCGCTCC; AGCGTCCC; AGCGCCCC; AACGTCCC; AACGCCCC; AACGTTCC; AACGCTCC; GGCGTTCC; GGCGCTCC; GGCGTCCC; GGCGCCCC; GACGCTCG; GACGTCCG; GACGCCCG; GACGTTCG; AGCGCTCG; AGCGTTCG; AGCGTCCG; AGCGCCCG; AACGTCCG; AACGCCCG; AACGTTCG; AACGCTCG; GGCGTTCG; GGCGCTCG; GGCGTCCG; GGCGCCCG. In some embodiments, the immunomodulatory polynucleotide comprises the sequence 5'-TGACTGTGAACGTTCGAGATGA-3' (SEQ ID NO:1).

In some embodiments, the ISS comprises any of the following sequences:
GACGCU; GACGUC; GACGUU; GACGUT; GACGTU; AGCGUU; AGCGCU; AGCGUC; AGCGUT; AGCGTU; AACGUC; AACGUU; AACGCU; AACGUT; AACGTU; GGCGUU; GGCGCU; GGCGUC; GGCGUT; GGCGTU.

In some embodiments, the ISS comprises any of the following sequences:
GABGCTCC; GABGTCCC; GABGTTCC; GABGCCCC; AGBGTTCC; AGBGCTCC; AGBGTCCC; AGBGCCCC; AABGTCCC; AABGCCCC; AABGTTCC; AABGCTCC; GGBGTTCC; GGBGCTCC; GGBGTCCC; GGBGCCCC; GABGCTCG; GABGTCCG; GABGCCCG; GABGTTCG; AGBGCTCG; AGBGTTCG; AGBGTCCG; AGBGCCCG; AABGTCCG; AABGCCCG; AABGTTCG; AABGCTCG; GGBGTTCG; GGBGCTCG; GGBGTCCG; GGBGCCCG; GABGCTBG; GABGTCBG; GABGCCBG; GABGTTBG; AGBGCTBG; AGBGTTBG; AGBGTCBG; AGBGCCBG; AABGTCBG; AABGCCBG; AABGTTBG; AABGCTBG; GGBGTTBG; GGBGCTBG; GGBGTCBG; GGBGCCBG, where B is 5-bromocytosine.

In some embodiments, the ISS comprises any of the following sequences:
GABGCUCC; GABGUCCC; GABGUTCC; GABGTUCC; GABGUUCC; AGBGUUCC; AGBGTUCC; AGBGUTCC; AGBGCUCC; AGBGUCCC; AABGUCCC; AABGUUCC; AABGUTCC; AABGTUCC; AABGCUCC; GGBGUUCC; GGBGUTCC; GGBGTUCC; GGBGCUCC; GGBGUCCC; GABGCUCG; GABGUCCG; GABGUUCG; GABGUTCG;GABGTUCG; AGBGCUCG; AGBGUUCG; AGBGUTCG; AGBGTUCG; AGBGUCCG; AABGUCCG; AABGUUCG; AABGUTCG; AABGTUCG; AABGCUCG; GGBGUUCG; GGBGUTCG; GGBGTUCG; GGBGCUCG; GGBGUCCG; GABGCUBG; GABGUCBG; GABGUUBG; GABGUTBG; GABGTUBG; AGBGCUBG; AGBGUUBG; AGBGUCBG; AGBGUTBG; AGBGTUBG; AABGUCBG; AABGUUBG; AABGUTBG; AABGTUBG; AABGCUBG; GGBGUUBG; GGBGUTBG; GGBGTUBG; GGBGCUBG; GGBGUCBG, where B is 5-bromocytosine.

In other embodiments, the ISS comprises any of the sequences:
5'-TGACCGTGAACGTTCGAGATGA-3' (SEQ ID NO:2);
5'-TCATCTCGAACGTTCCACAGTCA-3' (SEQ ID NO:3);
5'-TGACTGTGAACGTTCCAGATGA-3' (SEQ ID NO:4);
5'-TCCATAACGTTCGCCTAACGTTCGTC-3' (SEQ ID NO:5);
5'-TGACTGTGAABGTTCCAGATGA-3' (SEQ ID NO:6), where B is 5-bromocytosine;
5'-TGACTGTGAABGTTCGAGATGA-3' (SEQ ID NO:7), where B is 5-bromocytosine and
5'-TGACTGTGAABGTTBGAGATGA-3' (SEQ ID NO:8), where B is 5-bromocytosine.

An ISS and/or ISS-containing polynucleotide may contain modifications. Modifications of ISS include any known in the art, but are not limited to, modifications of the 3'-OH or 5'-OH group, modifications of the nucleotide base, modifications of the sugar component, and modifications of the phosphate group. Various such modifications are described below.

An ISS may be single stranded or double stranded DNA, as well as single or double-stranded RNA or other modified polynucleotides. An ISS may or may not include one or more palindromic regions, which may be present in the motifs described above or may extend beyond the motif. An ISS may comprise additional flanking sequences, some of which are described herein. An ISS may contain naturally-occurring or modified, non-naturally occurring bases, and may contain modified sugar, phosphate, and/or termini. For example, phosphate modifications include, but are not limited to, methyl phosphonate, phosphorothioate, phosphoramidate (bridging or non-bridging), phosphotriester and phosphorodithioate and may be used in any combination. Other non-phosphate linkages may also be used. Preferably, polynucleotides of the present invention comprise phosphorothioate backbones. Sugar modifications known in the field, such as 2'-alkoxy-RNA analogs, 2'-amino-RNA analogs and 2'-alkoxy- or amino-RNA/DNA chimeras and others described herein, may also be made and combined with any phosphate modification. Examples of base modifications include, but are not limited to, addition of an electron-withdrawing moiety to C-5 and/or C-6 of a cytosine of the ISS (*e.g*., 5-bromocytosine, 5-chlorocytosine, 5-fluorocytosine, 5-iodocytosine).

The ISS can be synthesized using techniques and nucleic acid synthesis equipment which are well known in the art including, but not limited to, enzymatic methods, chemical methods, and the degradation of larger polynucleotide sequences. See, for example, Ausubel et al. (1987); and Sambrook et al. (1989). When assembled enzymatically, the individual units can be ligated, for example, with a ligase such as T4 DNA or RNA ligase. U.S. Patent No. 5,124,246. Polynucleotide degradation can be accomplished through the exposure of an polynucleotide to a nuclease, as exemplified in U.S. Patent No. 4,650,675.

The ISS can also be isolated using conventional polynucleotide isolation procedures. Such procedures include, but are not limited to, hybridization of probes to genomic or cDNA libraries and synthesis of particular native sequences by the polymerase chain reaction.

Circular ISS can be isolated, synthesized through recombinant methods, or chemically synthesized. Where the circular ISS is obtained through isolation or through recombinant methods, the ISS will preferably be a plasmid. The chemical synthesis of smaller circular oligonucleotides can be performed using any method described in the literature. See, for instance, Gao et al. (1995) Nucleic Acids Res. 23:2025-2029; and Wang et al. (1994) Nucleic Acids Res. 22:2326-2333.

The techniques for making polynucleotides and modified polynucleotides are known in the art. Naturally occurring DNA or RNA, containing phosphodiester linkages, is generally synthesized by sequentially coupling the appropriate nucleoside phosphoramidite to the 5'-hydroxy group of the growing polynucleotide attached to a solid support at the 3'-end, followed by oxidation of the intermediate phosphite triester to a phosphate triester. Once the desired polynucleotide sequence has been synthesized, the polynucleotide is removed from the support, the phosphate triester groups are deprotected to phosphate diesters and the nucleoside bases are deprotected using aqueous ammonia or other bases. See, for example, Beaucage (1993) "Oligodeoxyribonucleotide Synthesis" in Protocols for Oligonucleotides and Analogs, Synthesis and Properties (Agrawal, ed.) Humana Press, Totowa, NJ; Warner et al. (1984) DNA 3:401 and U.S. Patent No. 4,458,066.

The ISS can also contain phosphate-modified polynucleotides. Synthesis of polynucleotides containing modified phosphate linkages or non-phosphate linkages is also known in the art. For a review, see Matteucci (1997) "Oligonucleotide Analogs: an Overview" in Oligonucleotides as Therapeutic Agents, (D.J. Chadwick and G. Cardew, ed.) John Wiley and Sons, New York, NY. The phosphorous derivative (or modified phosphate group) which can be attached to the sugar or sugar analog moiety in the polynucleotides of the present invention can be a monophosphate, diphosphate, triphosphate, alkylphosphonate, phosphorothioate, phosphorodithioate or the like. The preparation of the above-noted phosphate analogs, and their incorporation into nucleotides, modified nucleotides and polynucleotides, *per se,* is also known and need not be described here in detail. Peyrottes et al. (1996) Nucleic Acids Res. 24:1841-1848; Chaturvedi et al. (1996) Nucleic Acids Res. 24:2318-2323; and Schultz et al. (1996) Nucleic Acids Res. 24:2966-2973. For example, synthesis of phosphorothioate polynucleotides is similar to that described above for naturally occurring polynucleotides except that the oxidation step is replaced by a sulfurization step (Zon (1993) "Oligonucleoside Phosphorothioates" in Protocols for Oligonucleotides and Analogs, Synthesis and Properties (Agrawal, ed.) Humana Press, pp. 165-190). Similarly the synthesis of other phosphate analogs, such as phosphotriester (Miller et al. (1971) JACS 93:6657-6665), non-bridging phosphoramidates (Jager et al. (1988) Biochem. 27:7247-7246), N3' to P5' phosphoramidates (Nelson et al. (1997) JOC 62:7278-7287) and phosphorodithioates (U.S. Patent No. 5,453,496) has also been described. Other non-phosphorous based modified polynucleotides can also be used (Stirchak et al. (1989) Nucleic Acids Res. 17:6129-6141). Polynucleotides with phosphorothioate backbones can be more immunogenic than those with phosphodiester backbones and appear to be more resistant to degradation after injection into the host. Braun et al. (1988) J. Immunol. 141:2084-2089; and Latimer et al. (1995) Mol. Immunol. 32:1057-1064.

ISS-containing polynucleotides used in the invention can comprise ribonucleotides (containing ribose as the only or principal sugar component), deoxyribonucleotides (containing deoxyribose as the principal sugar component), or, as is known in the art, modified sugars or sugar analogs can be incorporated in the ISS. Thus, in addition to ribose and deoxyribose, the sugar moiety can be pentose, deoxypentose, hexose, deoxyhexose, glucose, arabinose, xylose, lyxose, and a sugar "analog" cyclopentyl group. The sugar can be in pyranosyl or in a furanosyl form. In the ISS, the sugar moiety is preferably the furanoside of ribose, deoxyribose, arabinose or 2'-0-alkylribose, and the sugar can be attached to the respective heterocyclic bases either in α or β anomeric configuration. Sugar modifications include, but are not limited to, 2'-alkoxy-RNA analogs, 2'-amino-RNA analogs and 2'-alkoxy- or amino-RNA/DNA chimeras. The preparation of these sugars or sugar analogs and the respective "nucleosides" wherein such sugars or analogs are attached to a heterocyclic base (nucleic acid base) *per se* is known, and need not be described here, except to the extent such preparation can pertain to any specific example. Sugar modifications may also be made and combined with any phosphate modification in the preparation of an ISS.

The heterocyclic bases, or nucleic acid bases, which are incorporated in the ISS can be the naturally-occurring principal purine and pyrimidine bases, (namely uracil or thymine, cytosine, adenine and guanine, as mentioned above), as well as naturally-occurring and synthetic modifications of said principal bases.

Those skilled in the art will recognize that a large number of "synthetic" non-natural nucleosides comprising various heterocyclic bases and various sugar moieties (and sugar analogs) are available in the art, and that as long as other criteria of the present invention are satisfied, the ISS can include one or several heterocyclic bases other than the principal five base components of naturally-occurring nucleic acids. Preferably, however, the heterocyclic base in the ISS includes, but is not limited to, uracil-5-yl, cytosin-5-yl, adenin-7-yl, adenin-8-yl, guanin-7-yl, guanin-8-yl, 4-aminopyrrolo (2.3-d) pyrimidin-5-yl, 2-amino-4-oxopyrolo (2,3-d) pyrimidin-5-yl, 2-amino-4-oxopyrrolo (2.3-d) pyrimidin-3-yl groups, where the purines are attached to the sugar moiety of the ISS via the 9-position, the pyrimidines via the 1-position, the pyrrolopyrimidines via the 7-position and the pyrazolopyrimidines via the 1-position.

The ISS may comprise at least one modified base as described, for example, in the commonly owned international application WO 99/62923. As used herein, the term "modified base" is synonymous with "base analog", for example, "modified cytosine" is synonymous with "cytosine analog." Similarly, "modified" nucleosides or nucleotides are herein defined as being synonymous with nucleoside or nucleotide "analogs." Examples of base modifications include, but are not limited to, addition of an electron-withdrawing moiety to C-5 and/or C-6 of a cytosine of the ISS. Preferably, the electron-withdrawing moiety is a halogen. Such modified cytosines can include, but are not limited to, azacytosine, 5-bromocytosine, bromouracil, 5-chlorocytosine, chlorinated cytosine, cyclocytosine, cytosine arabinoside, 5-fluorocytosine, fluoropyrimidine, fluorouracil, 5,6-dihydrocytosine, 5-iodocytosine, hydroxyurea, iodouracil, 5-nitrocytosine, uracil, and any other pyrimidine analog or modified pyrimidine.

The preparation of base-modified nucleosides, and the synthesis of modified polynucleotides using said base-modified nucleosides as precursors, has been described, for example, in U.S. Patents 4,910,300, 4,948,882, and 5,093,232. These base-modified nucleosides have been designed so that they can be incorporated by chemical synthesis into either terminal or internal positions of an polynucleotide. Such base-modified nucleosides, present at either terminal or internal positions of an polynucleotide, can serve as sites for attachment of a peptide or other antigen. Nucleosides modified in their sugar moiety have also been described (including, but not limited to, *e.g*., U.S. Patents 4,849,513, 5,015,733, 5,118,800, 5,118,802) and can be used similarly.

The ISS used in the methods of the invention may be produced as ISS-microcarrier complexes. ISS-microcarrier complexes comprise an ISS-containing polynucleotide bound to a microcarrier (MC). ISS-MC complexes comprise an ISS bound to the surface of a microcarrier (*i.e*., the ISS is not encapsulated in the MC), adsorbed within a microcarrier (*e.g*., adsorbed to PLGA beads), or encapsulated within a MC (*e.g*., incorporated within liposomes).

ISS-containing oligonucleotides bound to microparticles (SEPHAROSE® beads) have previously been shown to have immunostimulatory activity *in vitro* (Liang et al., (1996), J. Clin. Invest. 98:1119-1129). However, recent results show that ISS-containing oligonucleotides bound to gold, latex and magnetic particles are not active in stimulating proliferation of 7TD1 cells, which proliferate in response to ISS-containing oligonucleotides (Manzel et al., (1999), Antisense Nucl. Acid Drug Dev. 9:459-464).

Microcarriers are not soluble in pure water, and are less than about 50-60 µm in size, preferably less than about 10 µm in size, more preferably from about 10 nm to about 10 µm, 25 nm to about 5 µm, 50 nm to about 4.5 µm or 1.0 µm to about 2.0 µm in size. Microcarrers may be any shape, such as spherical, ellipsoidal, rod-shaped, and the like, although spherical microcarriers are normally preferred. Preferred microcarriers have sizes of or about 50 nm, 200 nm, 1 µm, 1.2 µm, 1.4 µm, 1.5 µm, 1.6 µm, 1.8 µm, 2.0 µm, 2.5 µm or 4.5 µm. The "size" of a microcarier is generally the "design size" or intended size of the particles stated by the manufacturer. Size may be a directly measured dimension, such as average or maximum diameter, or may be determined by an indirect assay such as a filtration screening assay. Direct measurement of microcarrier size is typically carried out by microscopy, generally light microscopy or scanning electron microscopy (SEM), in comparison with particles of known size or by reference to a micrometer. As minor variations in size arise during the manufacturing process, microcarriers are considered to be of a stated size if measurements show the microcarriers are + about 5-10% of the stated measurement. Size characteristics may also be determined by dynamic light scattering. Alternately, microcarrier size may be determined by filtration screening assays. A microcarrier is less than a stated size if at least 97% of the particles pass through a "screen-type" filter (i. e., a filter in which retained particles are on the surface of the filter, such as polycarbonate or polyethersulfone filters, as opposed to a "depth filter" in which retained particles lodge within the filter) of the stated size. A microcarrier is larger than a stated size if at least about 97% of the microcarrier particles are retained by a screen-type filter of the stated size. Thus, at least about 97% microcarriers of about 10 µm to about 10 nm in size pass through a 10 µm pore screen filter and are retained by a 10 nm screen filter.

As above discussion indicates, reference to a size or size range for a microcarrier implicitly includes approximate variations and approximations of the stated size and/or size range. This is reflected by use of the term "about" when referring to a size and/or size range, and reference to a size or size range without reference to "about" does not mean that the size and/or size range is exact.

Microcarriers may be solid phase (*e.g*., polystyrene beads) or liquid phase (*e.g*., liposomes, micelles, or oil droplets in an oil and water emulsion). Liquid phase microcarriers include liposomes, micelles, oil droplets and other lipid or oil-based particles. One preferred liquid phase microcarrier is oil droplets within an oil-in-water emulsion. Preferably, oil-in-water emulsions used as microcarriers comprise biocompatible substituents such as squalene. Liquid phase microcarriers are normally considered nonbiodegradable, but may be biodegradable liquid phase microcarriers may be produced by incorporation of one or more biodegradable polymers in the liquid microcarrier formulation. In one preferred embodiment, the microcarrier is oil droplets in an oil-in-water emulsion prepared by emulsification of squalene, sorbitan trioleate, TWEEN 80® in polyanhydrides such as poly(anhydride) polymers based on sebacic acid, p-(carboxyphenoxy)propane, or p-(carboxyphenoxy)hexane; polyanhydride imides, such as polyanhydride polymers based on sebacic acid-derived monomers incorporating amino acids (*i.e*., linked to sebacic acid by imide bonds through the amino-terminal nitrogen) such as glycine or alanine; polyanhydride esters; polyphosphazenes, especially poly(phosphazenes) which contain hydrolysis-sensitive ester groups which can catalyze degradation of the polymer backbone through generation of carboxylic acid groups (Schacht et al. (1996) Biotechnol. Bioeng. 1996:102); and polyamides such as poly(lactic acid-co-lysine). A wide variety of nonbiodegradable materials suitable for manufacturing microcarriers are also known, including, but not limited to polystyrene, polyethylene, latex, gold, and ferromagnetic or paramagnetic materials. Solid phase microcarriers may be covalently modified to incorporate one or more moieties for use in linking the ISS, for example by addition of amine groups for covalent linking using amine-reactive crosslinkers.

The ISS-microcarrier complexes of the invention may be covalently or non-covalently linked. Covalently linked ISS-MC complexes may be directly linked or be linked by a crosslinking moiety of one or more atoms (typically the residue of a crosslinking agent). The ISS may be modified to allow or augment binding to the MC (*e.g*., by incorporation of a free sulfhydryl for covalent crosslinking or addition of a hydrophobic moieties such as lipids, steroids, sterols such as cholesterol, and terpenes, for hydrophobic bonding), although unmodified ISS may be used for formation of non-covalent ISS-MC complex formation by electrostatic interaction or by base pairing (*e.g*., by base pairing at least one portion of the ISS with a complementary oligonucleotide bound to the microcarrier). ISS-containing polynucleotides may be linked to solid phase microcarriers or other chemical moieties to facilitate ISS-MC complex formation using conventional technology known in the art, such as use of available heterobifunctional crosslinkers (*e.g*., succinimidyl 4-(N-maleimidomethyl)cyclohexane-1-carboxylate or its sulfo-derivatives for covalently linking an amine-derivatized microcarrier and an ISS modified to contain a free sulfhydryl) or by addition of compounds such as cholesterol *(e.g.,* by the method of Godard et al. (1995) Eur. J. Biochem. 232:404-410) to facilitate binding to hydrophobic microcarriers such as oil droplets in oil-in-water emulsions. Alternatively, modified nucleosides or nucleotides, such as are known in the art, can be incorporated at either terminus, or at internal positions in the ISS. These can contain blocked functional groups which, when deblocked, are reactive with a variety of functional groups which can be present on, or attached to, the microcarrier or a moiety which would facilitate binding to a microcarrier. Certain embodiments of noncovalently linke ISS-MC complexes utilize a binding pair (*e.g*., an antibody and its cognate antigen or biotin and streptavidin or avidin), where one member of the binding pair is bound to the ISS and the microcarrier is derivatized with the other member of the binding pair (*e.g*., a biotinylated ISS and a streptavidin-derivatized microcarrier may be combined to form a noncovalently linked ISS-MC complex).

Non-covalent ISS-MC complexes bound by electrostatic binding typically exploit the highly negative charge of the polynucleotide backbone. Accordingly, microcarriers for use in non-covalently bound ISS-MC complexes are generally positively charged at physiological pH (*e.g*., about pH 6.8-7.4). The microcarrier may intrinsically possess a positive charge, but microcarriers made from compounds not normally possessing a positive charge may be derivatized or otherwise modified to become positively charged. For example, the polymer used to make the microcarrier may be derivatized to add positively charged groups, such as primary amines. Alternately, positively charged compounds may be incorporated in the formulation of the microcarrier during manufacture (*e.g*., positively charged surfactants may be used during the manufacture of poly(lactic acid)/poly(glycolic acid) copolymers to confer a positive charge on the resulting microcarrier particles.

Solid phase microspheres are prepared using techniques known in the art. For example, they can be prepared by emulsion-solvent extraction/evaporation technique. Generally, in this technique, biodegradable polymers such as polyanhydrates, poly(alkyl-α-cyanoacrylates) and poly(α-hydroxy esters), for example, poly(lactic acid), poly(glycolic acid), poly(D,L-lactic-co-glycolic acid) and poly(caprolactone), are dissolved in a suitable organic solvent, such as methylene chloride, to constitute the dispersed phase (DP) of emulsion. DP is emulsified by high-speed homogenization into excess volume of aqueous continuous phase (CP) that contains a dissolved surfactant, for example, polyvinylalcohol (PVA) or polyvinylpirrolidone (PVP). Surfactant in CP is to ensure the formation of discrete and suitably-sized emulsion droplet. The organic solvent is then extracted into the CP and subsequently evaporated by raising the system temperature. The solid microparticles are then separated by centrifugation or filtration, and dried, for example, by lyophilization or application of vaccum, before storing at 4 °C.

Generally, to prepare cationic microspheres, cationic lipids or polymers, for example, 1,2-dioleoyl-1,2,3-trimethylammoniopropane (DOTAP), cetyltrimethylammonium bromide (CTAB) or polylysine, are added either to DP or CP, as per their solubility in these phases.

Physico-chemical characteristics such as mean size, size distribution and surface charge of dried microspheres may be determined. Size characteristics are determined, for example, by dynamic light scattering technique and the surface charge was determined by measuring the zeta potential.

Generally, ISS-containing polynucleotides can be adsorbed onto the cationic microspheres by overnight aqueous incubation of ISS and the particles at 4 °C. Microspheres are characterized for size and surface charge before and after ISS association. Selected batches may then evaluated for activity as described herein.

### Administration

An ISS-containing polynucleotide may be administered before, during and/or after exposure to a papillomavirus. An ISS polynucleotide may also be administered before, during and/or after infection by a papillomavirus. An ISS-containing polynucleotide may also be administered before or after onset of symptoms of papillomavirus infection. An ISS-containing polynucleotide may also be administered before the development of papillomavirus-associated carcinoma (*i.e*., a pre-cancerous state). Accordingly, administration of ISS-containing polynucleotide may be at various times with respect to exposure to, infection by and/or onset of symptoms of infection by papillomavirus, Further, there may be one or more administrations. If the ISS-containing polynucleotide is administered on multiple occasions, the ISS may be administered on any schedule selected by the clinician, such as daily, every other day, every three days, every four days, every five days, every six days, weekly, biweekly, monthly or at ever longer intervals (which may or may not remain the same during the course of treatments). Where multiple administrations are given, the ISS-containing polynucleotide may be given in 2, 3, 4, 5, 6, 7, 8, 9, 10 or more separate administrations.

When ISS-containing polynucleotide is administered to an individual at risk of exposure to virus (*i.e*., before infection), ISS-containing polynucleotide is preferably administered less than about 14 days before exposure to virus, preferably less than about 10 days before exposure to virus, more preferably less than about 7 days before exposure to virus, even more preferably less than about 5 days before exposure to virus. In some embodiments, ISS-containing polynucleotide is administered about 3 days before exposure to virus.

In a further embodiment, the ISS-containing polynucleotide is administered after exposure to or infection by a papillomavirus, but prior to appearance of symptoms. This embodiment is particularly relevant with respect to HPV since years can elapse between exposure to papillomavirus and possible progression to carcinoma. Preferably, the ISS-containing polynucleotide is administered less than about three days after exposure, more preferably less than about one day, 12 hours, six hours or two hours after exposure, if the time of exposure is known or suspected.

In another embodiment, the ISS-containing polynucleotide is administered after appearance of at least one symptom of papillomavirus infection. Preferably, ISS-containing polynucleotide is administered within about 28, 21, 14, 7, 5 or 3 days following appearance of a symptom of papillomavirus infection. However, some infected individuals exhibiting symptoms will already have undertaken one or more courses of treatment with another therapy. In such individuals, or in individuals who failed to appreciate the import of their symptoms, the ISS-containing polynucleotide may be administered at any point following infection.

In another embodiment, the ISS-containing polynucleotide is administered after the appearance of at least one symptom of papillomavirus infection and preferably before the development of carcinoma. The staging of abnormal cellular growth (dysplasia) may be accomplished by any of the methods known in art, including, but not limited to, Pap smears, local biopsies, and *in situ* hybridization.

Additionally, treatments employing an ISS-containing polynucleotide may also be employed in conjunction with other treatments or as 'second line' treatments employed after failure of a 'first line' treatment (*e.g*., ISS-containing polynucleotide therapy may be employed in conjunction with physical removal and/or cryogenic treatment of papillomavirus-induced lesions).

ISS polynucleotides may be formulated in any form known in the art, such as dry powder, semi-solid or liquid formulations. For parenteral administration ISS polynucleotides preferably administered in a liquid formulation, although solid or semi-solid formulations may also be acceptable, particularly where the ISS polynucleotide is formulated in a slow release depot form. ISS polynucleotides are generally formulated in liquid or dry powder form for topical administration, although semi-solid formulations may occasionally be useful.

ISS polynucleotide formulations may contain additional components such as salts, buffers, bulking agents, osmolytes, antioxidants, detergents, surfactants and other pharmaceutically-acceptable excipients as are known in the art. Generally, liquid ISS polynucleotide formulations made in USP water for injection and are sterile, isotonic and pH buffered to a physiologically-acceptable pH, such as about pH 6.8 to 7.5.

ISS-containing polynucleotides may be formulated in delivery vehicles such as liposomes, oil/water emulsion or slow release depot formulations. Methods of formulating polynucleotides in such forms are well known in the art.

ISS-containing polynucleotide formulations may also include or exclude immunomodulatory agents such as adjuvants and immunostimulatory cytokines, which are well known in the art.

A suitable dosage range or effective amount is one that provides the desired reduction of symptoms and/or suppression of viral infection and depends on a number of factors, including the particular papillomavirus, ISS sequence of the polynucleotide, molecular weight of the polynucleotide and route of administration. Dosages are generally selected by the physician or other health care professional in accordance with a variety of parameters known in the art, such as severity of symptoms, history of the patient and the like. Generally, for an ISS-containing polynucleotide of about 20 bases, a dosage range may be selected from, for example, an independently selected lower limit such as about 0.1, 0.25, 0.5, 1, 2, 5, 10, 20, 30 40, 50 60, 80, 100, 200, 300, 400 or 500 µg/kg up to an independently selected upper limit, greater than the lower limit, of about 60, 80, 100, 200, 300, 400, 500, 750, 1000, 1500, 2000, 3000, 4000, 5000, 6000, 7000, 8000, 9000 or 10,000 µg/kg. For example, a dose may be about any of the following: 0.1 to 100 µg/kg, 0.1 to 50 µg/kg, 0.1 to 25 µg/kg, 0.1 to 10 µg/kg, 1 to 500 µg/kg, 100 to 400 µg/kg, 200 to 300 µg/kg, 1 to 100 µg/kg, 100 to 200 µg/kg, 300 to 400 µg/kg, 400 to 500 µg/kg, 500 to 1000 µg/kg, 500 to 5000 µg/kg, or 500 to 10,000 µg/kg. Generally, parenteral routes of administration require higher doses of ISS compared to more direct application to infected tissue, as do ISS-containing polynucleotides of increasing length.

Polynucleotides comprising an ISS may be administered by systemic (*e.g*., parenteral) or local (*e.g*., topical or intralesional injection) administration.

In one embodiment, the ISS-containing polynucleotide(s) is topically administered. Topical administration may be at the site of infection (*e.g*., genital region in the case of mucosal papillomavirus), it may be at a site of a symptom (*e.g*., a papilloma lesion) or it may be at the site of possible exposure to papillomavirus (*e.g*., gential region).

In another embodiment, the ISS-containing polynucleotide(s) is injected locally into the area of lesion(s). Intralesional injection may be at the site of infection (*e.g*., genital region in the case of mucosal papillomavirus), site of dysplasia (*e.g*., epithelium in the genital region) or it may be at a site of a symptom (*e.g*., a papilloma lesion).

In other embodiments, the ISS-containing polynucleotide is administered parenterally. Parenteral routes of administration include, but are not limited to, transdermal, transmucosal, nasopharyngeal, pulmonary and direct injection. Parenteral administration by injection may be by any parenteral injection route, including, but not limited to, intravenous (IV), intraperitoneal (IP), intramuscular (IM), subcutaneous (SC) and intradermal (ID) routes. Transdermal and transmucosal administration may be accomplished by, for example, inclusion of a carrier (*e.g*., dimethylsulfoxide, DMSO), by application of electrical impulses (*e.g*., iontophoresis) or a combination thereof. A variety of devices are available for transdermal administration which may be used in accordance with the invention.

Nasopharyngeal and pulmonary routes of administration include; but are not limited to, intranasal, inhalation, transbronchial and transalveolar routes. The ISS-containing polynucleotide may thus be administered by inhalation of aerosols, atomized liquids or powders. Devices suitable for administration by inhalation of ISS-containing compositions include, but are not limited to, nebulizers, atomizers, vaporizers, and metered-dose inhalers. Nebulizers, atomizers, vaporizers and metered-dose inhalers filled with or employing reservoirs containing formulations comprising the ISS-containing polynucleotide(s) are among a variety of devices suitable for use in inhalation delivery of the ISS-containing polynucleotide(s). Other methods of delivering to respiratory mucosa include delivery of liquid formulations, such as by nose drops.

IV, IP, IM and ID administration may be by bolus or infusion administration. For SC administration, administration may be by bolus, infusion or by implantable device, such as an implantable minipump (*e.g*., osmotic or mechanical minipump) or slow release implant. The ISS polynucleotide(s) may also be delivered in a slow release formulation adapted for IV, IP, IM, ID or SC administration. Administration by inhalation is preferably accomplished in discrete doses (*e.g*., via a metered dose inhaler), although delivery similar to an infusion may be accomplished through use of a nebulizer. Administration via the transdermal and transmucosal routes may be continuous or pulsatile.

### Assessment

In some embodiments, administration of an ISS-containing polynucleotide results in prevention, palliation and/or improvement in one or more symptoms of papillomavirus infection. The exact form of prevention, palliation or improvement will depend on the particular *papillomavirinae* type and the symptoms experienced by the individual but includes reduction in size and/or duration of lesions and/or warts, reduction in symptoms of papillomavirus infection or reduction in frequency or number of recurrent lesions. In some embodiments, administration of an ISS-containing polynucleotide results in a reduction in viral titer (a reduction of which indicates suppression of viral infection). In other embodiments, the number of warts is reduced. In other embodiments, viral infection is suppressed, which may be indicated by any one or more of a number of parameters, including, but not limited to, extent of one or more symptoms and viral titer. In other embodiments, recurrence, which is generally indicated by appearance of one or more symptoms associated with infection, is reduced.

Symptoms of infection may be assessed before or after administration of ISS-containing polynucleotide by the individual or the clinician. As will be apparent to one of skill in the art, the symptoms will vary depending on the particular papillomavirus (*e.g*., cutaneous or mucosal, high-risk or low-risk) and the site of the symptoms (*e.g*., genital region, oral cavity, respiratory tract, skin, etc.). Symptoms of papillomavirus infection can include papilloma lesions on cutaneous and/or mucosal membranes, thickening of epithelial layer, nuclear changes such as enlargement, hyperchromasia, and/or pyknosis. Characteristics of papillomavirus lesions can include localized epithelial hyperplasia with a defined boundary, intact basement membrane, and differentiated epithelium. Additional characteristics may include koilocytosis, large perinuclear cavitation with irregular edges and dense cytoplasm in the area surrounding the cavity.

Viral titer may be assessed in biological samples using standard methods of the art. Levels of viral nucleic acid may be assessed by isolating nucleic acid from the sample and/or performing PCR analysis using virus specific primers or blot analysis using a viral polynucleotide sequence as a probe. The PCR analysis may be quantitative using PCR technology known in the art. Another method is to perform *in situ* hybridization with virus-specific probes. Another assay measures infectious units, such as infectious center assay (ICA). Extent or amount of viral particles may be measured from any infected area, such as infected tissue or mucosal discharge. When the sample is a liquid, viral titer is calculated in some indication of number or amount of virus or virus particles (*e.g*., infectious particles, plaque forming units, infectious doses, or median tissue culture infectious doses (TCID 50)) per unit volume. In solid samples, such as a tissue sample, viral titer is calculated in virus particles per unit weight. Reduction is indicated by comparing viral titer to viral titer measured at an earlier time point, and/or comparing to an estimated titer (based, for example, on animal or clinical studies) that represents untreated infection.

Abnormal cell growth in the cervix may be examined and classified into various stages according to procedures commonly practiced by clinicians. Samples obtained by methods such as Pap smears or local biopsy may be examined for morphological abnormalities. Abnormal cell growth in the cervix may be classified as either low-grade squamous intraepithelial lesion (SIL) or high-grade SIL. The category of low-grade SIL includes HPV infection and cervical intraepithelial neoplasia (CIN) 1 while high-grade SIL includes CIN 2 and 3 when the entire thickness of the epithelium is replaced by abnormal cells. This classification scheme is equivalent to a previous classification scheme in which the different stages ranged from cervical intraepithelial neoplasia (CIN) 1 (mild dysplasia) to CIN 2 (moderate dysplasia) to CIN 3 (carcinoma *in situ*)*.* Individuals with HPV infection but have not progressed to CIN1 are suitable candidates for the administration of ISS to prevent and/or reduce the chances of progression to carcinoma.

### Kits of the Invention

The invention provides kits for carrying out the methods of the invention. Accordingly, a variety of kits are provided. The kits may be used for any one or more of the following (and, accordingly, may contain instructions for any one or more of the following uses): preventing symptoms *of papillomavirinae* infection in an individual who has been exposed to *papillomavirinae;* preventing symptoms of *papillomavirinae* infection in an individual at risk of being exposed to *papillomavirinae;* reducing severity of a symptom of *papillomavirinae* infection in an individual infected with *papillomavirinae;* reducing recurrence of a symptom of *papillomavirinae* infection in an individual infected with *papillomavirinae;* delaying development of a *papillomavirinae* infection and/or a symptom of *papillomavirinae* infection in an individual infected or at risk of being infected with *papillomavirinae;* reducing duration of a *papillomavirinae* infection in an individual infected or at risk of being infected with *papillomavirinae.* As is understood in the art, any one or more of these uses would be included in instructions directed to treating or preventing *papillomavirinae* infection.

The kits of the invention comprise one or more containers comprising an ISS-containing polynucleotide and a set of instructions, generally written instructions although electronic storage media (*e.g*., magnetic diskette or optical disk) containing instructions are also acceptable, relating to the use and dosage of the ISS-containing polynucleotide for the intended treatment (*e.g*., preventing one or more symptoms *of papillomavirinae* infection in an individual who has been exposed to *papillomavirinae;* preventing one or more symptoms of *papillomavirinae* infection in an individual who is at risk of being exposed to *papillomavirinae;* reducing severity of a symptom of *papillomavirinae* infection in an individual infected with *papillomavirinae;* and/or reducing recurrence of one or more symptoms *of papillomavirinae* infection in an individual infected with *papillomavirinae*)*.* The instructions included with the kit generally include information as to dosage, dosing schedule, and route of administration for the intended treatment. The containers of ISS may be unit doses, bulk packages (*e.g*., multi-dose packages) or sub-unit doses.

The kits of the invention do not include any packages or containers which include viral antigens from the *papillomavirinae* the kit is intended to be used to treat. Accordingly, neither the container comprising the ISS-containing polynucleotide nor any other containers in the kit contain *papillomavirinae* viral antigens.

The ISS component of the kit may be packaged in any convenient, appropriate packaging. For example, if the ISS is a freeze-dried formulation, a vial with a resilient stopper is normally used, so that the drug may be easily reconstituted by injecting fluid through the resilient stopper. Ampoules with non-resilient, removable closures (*e.g*., sealed glass) or resilient stoppers are most conveniently used for injectable forms of ISS. Also, prefilled syringes may be used when the kit is supplied with a liquid formulation of the ISS-containing polynucleotide. The kit may contain the ISS in an ointment for topical formulation in appropriate packaging. Also contemplated are packages for use in combination with a specific device, such as an inhaler, nasal administration device (*e.g*., an atomizer), transdermal administration device, or an infusion device such as a minipump.

As stated above, any ISS-containing polynucleotide described herein may be used, such as, for example, any polynucleotide comprising any of the following ISS: the sequence 5'-C, G, pyrimidine, pyrimidine, C, G-3', the sequence 5'-purine, purine, C, G, pyrimidine, pyrimidine, C, G-3', the sequence 5'-purine, purine, C, G, pyrimidine, pyrimidine, C, C-3'; the sequence SEQ ID NO: 1; the sequence 5'-urine, purine, B, G, pyrimidine, pyrimidine-3' wherein B is 5-bromocytosine or the sequence 5'-purine, purine, B, G, pyrimidine, pyriinidine, C, G-3' wherein B is 5-bromocytosine.

The following Examples are provided to illustrate, but not limit, the invention.

### EXAMPLES

### Example 1: Treatment of Canine Oral Papilloma with ISS

A model of canine oral papilloma was used to test the efficacy of ISS on papilloma. Beagle puppies were inoculated in the bucal mucosa with canine papillomavirus and developing papilloma lesions were monitored daily. Four groups of seven dogs each were treated with differing amount of ISS oligonucleotide (5'-TGACTGTGAACGTTCGAGATGA-3' (SEQ ID NO:1), phosphorothioate backbone). One group received 50 µg ISS twice a week, another group received 500 µg ISS twice a week, the third group received 500 µg ISS one time only at the first signs of papilloma lesion development (injected within the papilloma lesion) and the fourth group (control group) received PBS twice a week. All dogs were monitored daily for the development of lesions and the time to regression.

The results are shown in Figure 1: Dogs that received a one time treatment of 500 µg ISS at the first signs of papilloma lesion showed a higher average rate of lesion regression than untreated dogs, although the ranges for both groups overlapped. Untreated dogs took an average of 29.1 days for rapid regression while dogs treated with 500 µg ISS at the first signs of papilloma took an average of 25.1 days for rapid regression.

The other treatment groups did not show a marked difference in regression time. This model offers a short window of time in which regression of warts can be observed. In dogs, warts caused by canine papillomaviruses can spontaneously regress. Injection of ISS in the papillomas when papillomas first appear appears to enhance the time of lesion regression as compared to the time of spontaneous lesion regression.

### Example 2: Treatment of cutaneous papillomatosis in a rabbit model by ISS :

Rabbits were initially the first animals in which papillomavirus infection was described in 1933 by Shope. Shope recognized the cottontail rabbit papillomavirus (CRPV) as the etiological agent for cutaneous papillomatosis in the cottontail rabbit (Howley, P., Chapter 65, Fields Virology, Vol. 2, Third Edition, Lippincott-Raven publishers).

In this model of papilloma, New Zealand White rabbits of both genders were quarantined for 14 days, those animals remaining healthy were cleared for use in the experiment. 15 rabbits were each inoculated with a high dose of CRPV at two different sites (one on each side fo the animal) and a low dose of CRPV at two different sites (one on each side fo the animal) for a total of four inoculation sites in each rabbit. The animals were then separated into three groups of five animals each, groups A, B, and C.

Group A received 50 µg intradermal injections of ISS oligonucleotide (5'-TGACTGTGAACGTTCGAGATGA-3' (SEQ ID NO:1), phosphorothioate backbone) into the site of CRPV inoculation (site of the papilloma lesion at later time points) at Day 1 (one day following inoculation with CRPV) and Day 21 on the left side and at Day 14 and Day 35 on the right side. Groups B and C received intradermal injections of 500 µg of the ISS and phosphate-buffered saline (vehicle), respectively, into the site of CRPV inoculation (site of the papilloma lesion at later time points) on the same schedule.

Papilloma development was quantitated by finding the geometric mean diameter (GMD) of each papilloma lesion. GMD was calculated from measurements of the length, width and height of the papilloma lesions. Measurements were made weekly.

Results are summarized in Figure 2. Panel A shows GMD for the left side, high CRPV dose lesions (treatment on Day 1 and 14). Panel B shows GMD for the left side, low CRPV dose lesions (treatment on Day 14 and 35). Panel C shows average GMD for the right side, high CRPV dose lesions (treatment on Day 1 and 14). Panel D shows average GMD for the right side, low CRPV dose lesions (treatment on Day 14 and 35).

In another experiment, a mutant of CRPV which induces small papillomas, CRPV-E8m, was used to induce papillomas on five rabbit treatment groups (five rabbits per group). In each animal, papillomas on the left side of the animal received treatments and papillomas on the right side were untreated. Four of the treatment groups received doses between 100 µg and 2000 µg of ISS as intradermal injections per papilloma at several treatment regimes and the fifth group received injections of PBS as control, as outlined below.

| Group | Left Side Treatment |
|---|---|
| A | ISS; 100 µg/injection; 3 times/week from days 47 - 86 |
| B | ISS; 100 µg/injection; 1 time/week from days 47 - 86 |
| C | ISS; 500 µg/injection; 1 time/week from days 47 - 86 |
| D | ISS; 2000 µg/injection; weeks 7 and 10 |
| E | PBS; 100 µl/injection; 3 times/week from days 47 - 86 |

Four papillomas, initiated with CRPV-E8m plasmid DNA, were established on each rabbit. Skin at the site of papilloma initiation was made hyperplastic using a mixture of turpentine and acetone prior to viral DNA administration. The size of papillomas was measured (three dimensions, in mm) and the GMD calculated for each papilloma.

In this experiment, a number of viral DNA challenged sites failed to generate any papillomas. Minimal differences were found in the papilloma growth rates of the treated versus untreated papillomas for Treatment Groups A, B, D and E. Results from Treatment Group C are depicted in Figure 3 and demonstrate a reduction in the size of the ISS treated papillomas compared to untreated papillomas.

The present invention has been detailed both by direct description and by example.

## Claims

1. A composition comprising a polynucleotide comprising an immunostimulatory sequence (ISS), for use in a method for treating a symptom of papillomavirus infection in an individual who has been exposed to papillomavirus, wherein the ISS comprises the sequence 5'-C,G,pyrimidine,pyrimidine,C,G-3' and the polynucleotide is 8 to 50 base or base pairs in length, wherein the polynucleotide is administered without co-administration of a papillomavirus antigen, wherein said composition does not comprise a cytokine and wherein said composition is for administration in an amount sufficient to treat a symptom of papillomavirus infection, and wherein said composition is to be administered at a site of a papilloma lesion.

2. The composition for use according to claim 1, wherein the ISS comprises the sequence 5'-CGTTCG-3'.

3. The composition for use according to claim 1, wherein the ISS comprises the sequence 5'-purine,purine,C,G,pyrimidine,pyrimidine,C,G-3'.

4. The composition for use according to claim 1, wherein the ISS comprises a sequence selected from the group consisting of 5'-AACGTTCG-3', and 5'-GACGTTCG-3'.

5. The composition for use according to claim 1, wherein the ISS comprises the sequence 5'-TGACTGTGAACGTTCGAGATGA-3' (SEQ ID NO: 1).

6. The composition for use according to any one of the preceding claims, wherein the individual is a mammal.

7. The composition for use according to any one of the preceding claims, wherein the papillomavirus is a human papillomavirus (HPV).

8. The composition for use according to any one of claims 1 to 6, wherein the papillomavirus is an animal papillomavirus.

## Patentansprüche

1. Zusammensetzung, umfassend ein Polynukleotid, welches eine immunstimulatorische Sequenz (ISS) umfasst, zur Verwendung bei einer Methode zur Behandlung eines Symptoms einer Papillomvirus-Infektion in einem Individuum, das einem Papillomvirus ausgesetzt gewesen ist, wobei die ISS die Sequenz 5'-C,G,Pyrimidin,Pyrimidin,C,G-3' umfasst und das Polynukleotid 8 bis 50 Basen oder Basenpaare in der Länge beträgt, wobei das Polynukleotid ohne gleichzeitige Verabreichung eines Papillomvirus-Antigens verabreicht wird, wobei die Zusammensetzung kein Zytokin umfasst und wobei die Zusammensetzung zur Verabreichung in einer ausreichenden Menge ist, um ein Symptom einer Papillomvirus-Infektion zu behandeln, und wobei die Zusammensetzung an eine Stelle einer papillären Läsion zu verabreichen ist.

2. Zusammensetzung zur Verwendung nach Anspruch 1, wobei die ISS die Sequenz 5'-CGTTCG-3' umfasst.

3. Zusammensetzung zur Verwendung nach Anspruch 1, wobei die ISS die Sequenz 5'-Purin,Purin,C,G,Pyrimidin,Pyrimidin,C,G-3' umfasst.

4. Zusammensetzung zur Verwendung nach Anspruch 1, wobei die ISS eine Sequenz, ausgewählt aus der Gruppe, bestehend aus 5'-AACGTTCG-3' und 5'-GACGTTCG-3', umfasst.

5. Zusammensetzung zur Verwendung nach Anspruch 1, wobei die ISS die Sequenz 5'-TGACTGTGAACGTTCGAGATGA-3' (SEQ ID NR. 1) umfasst.

6. Zusammensetzung zur Verwendung nach einem der vorangehenden Ansprüche, wobei das Individuum ein Säuger ist.

7. Zusammensetzung zur Verwendung nach einem der vorangehenden Ansprüche, wobei das Papillomvirus ein humanes Papillomvirus (HPV) ist.

8. Zusammensetzung zur Verwendung nach einem der Ansprüche 1 bis 6, wobei das Papillomvirus ein animales Papillomvirus ist.

## Revendications

1. Composition comprenant un polynucléotide comprenant une séquence immunostimulatrice (ISS), pour utilisation dans un procédé de traitement d'un symptôme d'infection par papillomavirus chez un sujet qui a été exposé à un papillomavirus, dans laquelle l'ISS comprend la séquence 5'-C,G,pyrimidine, pyrimidine,C,G-3' et le polynucléotide a une longueur de 8 à 50 bases ou paires de bases, dans laquelle le polynucléotide est administré sans co-administration d'un antigène de papillomavirus, dans laquelle ladite composition ne comprend pas une cytokine et dans ladite composition est pour l'administration en une quantité suffisante pour traiter un symptôme d'infection par papillomavirus, et dans laquelle ladite composition doit être administrée au niveau d'un site de lésion à papillome.

2. Composition pour utilisation selon la revendication 1, dans laquelle l'ISS comprend la séquence 5'-CGTTCG-3'.

3. Composition pour utilisation selon la revendication 1, dans laquelle l'ISS comprend la séquence 5'-purine,purine,C,G,pyrimidine,pyrimidine,C,G-3'.

4. Composition pour utilisation selon la revendication 1, dans laquelle l'ISS comprend une séquence choisie parmi le groupe consistant en 5'-AACGTTCG-3' et 5'-GACGTTCG-3'.

5. Composition pour utilisation selon la revendication 1, dans laquelle l'ISS comprend la séquence 5'-TGACTGTGAACGTTCGAGATGA-3' (SEQ ID N° 1).

6. Composition pour utilisation selon l'une quelconque des revendications précédentes, dans laquelle le sujet est un mammmifère.

7. Composition pour utilisation selon l'une quelconque des revendications précédentes, dans laquelle le papillomavirus est un papillomavirus humain (HPV).

8. Composition pour utilisation selon l'une quelconque des revendications 1 à 6, dans laquelle le papillomavirus est un papillomavirus animal.
